# DEMANDE DE BREVET EUROPEEN

(11) **EP 0 643 046 A1**
(43) Date de publication de la demande: **15.03.1995**
(21) Numéro de dépôt: 94401962.9
(22) Date de dépôt: 05.09.1994
(51) Int. Cl.: C07D 215/36, A61K 31/47

(54) **Acides et chlorures d'acides 1,2,3,4-tétrahydroquinoléine-8-sulfoniques, comme intermédiaires**

(30) Priorité: 14.09.1993 FR 9310905
(71) Demandeur: SYNTHELABO, F-92350 Le Plessis Robinson (FR)
(72) Inventeur: Galtier Daniel, F-78280 Guyancourt (FR); Lassalle, Gilbert, F-92140 Clamart (FR)
(74) Mandataire: Thouret-Lemaitre, Elisabeth

(57) **Abrégé**

Dérivés optiquement purs répondant à la formule (I) 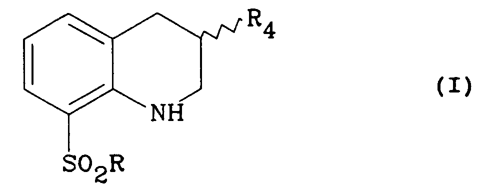
dans laquelle
R représente soit un atome de chlore soit un groupe hydroxy et
R₄ représente un groupe (C₁-C₄)alkyle.

Intermédiaires de synthèse.

## Description

La présente invention a pour objet des dérivés d'acides et de chlorures d'acides 1,2,3,4-tétrahydroquinoléine-8-sulfoniques optiquement purs, leur préparation et leur utilisation comme intermédiaires de synthèse.

Les composés de l'invention répondent à la formule (I) 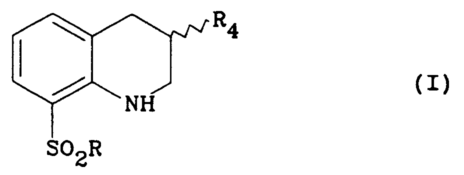
dans laquelle
R représente soit un atome de chlore soit un groupe hydroxy et
R₄ représente un groupe (C₁-C₄)alkyle.

Certains composés de formule (I) sous forme de racémate sont décrits à titre d'intermédiaires dans la demande de brevet européen EP 0565396.

Les composés de l'invention peuvent être préparés selon le procédé décrit dans le schéma 1 de la page suivante.
On fait réagir un composé de formule (II) dans laquelle R₄ est tel que défini ci-dessus et R₅ représente un groupe (C₁-C₄)alkyle avec du 1-iodo-2-nitrobenzène de formule (III) en présence d'un mélange de zinc et de cuivre et de diméthylacétamide comme catalyseurs, dans un solvant tel que le benzène. On obtient un composé de formule (IV) que l'on soumet à une hydrogénation catalytique à 50 psi en présence d'un catalyseur tel que l'oxyde de platine pour obtenir une dihydroquinoléine de formule (V). Ensuite on fait réagir du borane sur le composé de formule (V) ainsi obtenu dans un solvant tel que par exemple le tétrahydrofurane pour obtenir une tétrahydroquinoléine de formule (VI) sur laquelle on fait réagir du chlorure de chlorocarbonylsulfényle dans un solvant tel que par exemple le toluène et on obtient un composé de 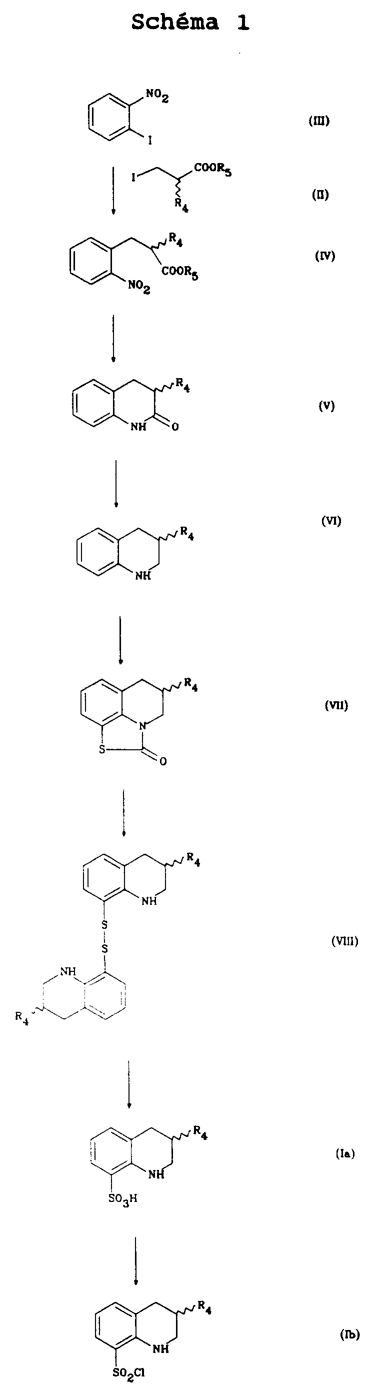
formule (VII) que l'on traite par de la potasse alcoolique puis par de l'alumine pour obtenir un composé de formule (VIII). Enfin, on traite le composé de formule (VIII) par de l'eau oxygénée en présence d'acide sulfurique pour obtenir l'acide sulfonique de formule (Ia).
Pour obtenir le chlorure de l'acide de formule (Ib), on fait réagir l'acide de formule (Ia) sur du chlorure de sulfuryle en présence de triphénylphosphine et d'une base comme la triéthylamine dans un solvant tel que par exemple le dichlorométhane.

Dans une variante du procédé, on peut préparer le composé de formule (V) en faisant réagir un composé de formule (II) avec de la 2-iodoaniline et du diméthylacétamide comme catalyseur et en présence de zinc et de cuivre dans un solvant tel que par exemple le benzène.

Pour obtenir les composés optiquement purs, on met en oeuvre le procédé de l'invention décrit dans le schéma 1 en utilisant les composés de formule (II) optiquement purs.

Les composés de départ sont disponibles dans le commerce ou décrits dans la littérature ou peuvent être préparés selon des méthodes qui y sont décrites ou qui sont connues de l'homme du métier.
Ainsi le (*R*)-3-iodo-2-méthylpropanoate de méthyle est préparé à partir du (*S*)-3-hydroxy-2-méthylpropanoate de méthyle selon la méthode décrite par Nakamura et coll. dans Tetrahedron Letters, (1987), 28, no 3, 337-340.

Les exemples qui suivent illustrent en détail la préparation des composés selon l'invention.
Les microanalyses et les spectres IR et RMN confirment la structure des composés obtenus.

### Exemple 1

acide (*S*)-3-méthyl-1,2,3,4-tétrahydroquinoléine-8-sulfonique
1.1. (*S*)-α-méthyl-2-nitrobenzènepropanoate de méthyle
   Sous atmosphère d'azote et sous agitation, on place 9,5 g (42 mmoles) de (*R*)-3-iodo-2-méthylpropanoate de méthyle dans 90 ml de benzène contenant 4,4 g du couple Zn(Cu) et 5,5 ml de diméthylacétamide. On agite pendant 15 minutes à la température ambiante puis on chauffe à 60 °C pendant 3 heures. Ensuite on laisse revenir le mélange à la température ambiante et on ajoute 1 g d'acétate de bis(tri-O-tolylphosphine) palladium en suspension dans 2 ml de benzène puis 7,5 g (30 mmoles) de 1-iodo-2-nitrobenzène en solution dans 20 ml de benzène. On chauffe, on laisse à 60 °C pendant 1 heure, on ajoute 100 ml d'acétate d'éthyle puis on filtre le milieu réactionnel sur célite. On lave le filtrat par 100 ml d'un solution 1 N d'acide chlorhydrique puis par 100 ml d'eau et on le sèche sur sulfate de magnésium. On purifie le résidu par chromatographie sur colonne de gel de silice en éluant par un mélange hexane/acétate d'éthyle (90/10).
   On obtient 3 g de produit sous forme d'une huile que l'on utilisera telle quelle dans l'étape suivante.
1.2. (*S*)-3-méthy1-3,4-dihydroquinoléin-2(1*H*)-one
   *Méthode 1*
      Dans 40 ml de méthanol on place 3 g (14 mmoles) du produit obtenu dans l'étape précédente et on ajoute 100 mg d'oxyde de platine. On hydrogène sous une pression de 50 psi dans un appareil de Parr pendant 8 heures puis on filtre, on essore le catalyseur et on évapore le filtrat à sec. On récupère le résidu et on le triture dans l'éther. On le recristallise dans l'éther éthylique.
      On obtient 1,5 g de produit sous forme de cristaux
      Point de fusion = 117-119 °C
   *Méthode 2*
      Sous atmosphère d'azote et sous agitation, on place 31 g (135 mmoles) de (*R*)-3-iodo-2-méthylpropanoate de méthyle dans 310 ml de benzène contenant 19,75 g du couple Zn(Cu) et 20,3 ml de diméthylacétamide. On chauffe le mélange à 60 °C pendant 3 heures puis on le refroidit à la température ambiante. On ajoute 2,92 g d'acétate de bis(tri-O-tolylphosphine)palladium en une seule fois puis 19,75 g (90 mmoles) de 2-iodoaniline en solution dans 50 ml de benzène. On chauffe pendant 1 heure à 50 °C puis on laisse revenir le mélange à la température ambiante. On filtre sur célite, on lave par 2 fois 100 ml d'acétate d'éthyle et on rassemble les filtrats. On les lave successivement par 2 fois 100 ml d'une solution 1 N d'acide chlorhydrique, par 100 ml d'une solution saturée d'hydrogénocarbonate de sodium et par 50 ml d'une solution saturée de chlorure de sodium. On sèche sur sulfate de magnésium et on évapore à sec. On purifie le résidu par chromatographie sur colonne de gel de silice en éluant par du dichlorométhane.
      On obtient 4,9 g de produit.
      Point de fusion = 118-120 °C
1.3. (*S*)-3-méthyl-1,2,3,4-tétrahydroquinoléine
   Dans 6 ml de tétrahydrofurane, on place 0,8 g (5 mmoles) du composé obtenu dans l'étape précédente et on ajoute, à 0 °C sous atmosphère d'azote, 17,5 ml d'une solution 1 M de borane dans le tétrahydrofurane. On chauffe à reflux pendant 1 heure et on ajoute lentement 5 ml d'eau. Ensuite on ajuste le pH du milieu réactionnel à 1 avec une solution 1 N d'acide chlorhydrique puis on chauffe au reflux pendant 2 heures. On laisse refroidir le mélange, on évapore, on reprend le résidu par 50 ml d'acétate d'éthyle et on le lave par 2 fois 50 ml d'une solution d'hydrogénocarbonate de sodium. On le sèche sur sulfate de magnésium et on évapore à sec. On purifie le résidu ainsi obtenu par chromatographie sur colonne de gel de silice en éluant par du dichlorométhane.
   On obtient 0,6 g de produit sous forme d'une huile que l'on utilisera telle quelle dans l'étape suivante.
   [α]_{D} = + 79 ° (c = 3, méthanol)
1.4. (*S*)-5-méthyl-5,6-dihydro-2*H*,4*H*-thiazolo[5,4,3-*ij*] quinoléin-2-one
   Sous atmosphère d'azote et sous agitation, on place 40,5 ml de toluène puis 2,28 ml (27 mmoles) de chlorure de chlorocarbonylsulfényle. On refroidit le milieu réactionnel à -50 °C puis on ajoute lentement un mélange de 3,5 g (23 mmoles) du composé obtenu dans l'étape précédente et de 3,24 g (28 mmoles) de *N*,*N*-diméthylbenzèneamine en solution dans 130 ml de toluène. On laisse le mélange remonter à la température ambiante, on ajoute 100 ml de toluène et on chauffe à 80 °C pendant 3 heures. On laisse refroidir et on lave par 2 fois 100 ml d'une solution 1 N d'acide chlorhydrique puis par 100 ml d'une solution saturée de NaCl. On sèche sur sulfate de magnésium puis on évapore à sec. On purifie le résidu ainsi obtenu par chromatographie sur colonne de gel de silice en éluant par du dichlorométhane.
   On obtient 4,3 g de produit.
   Point de fusion = 54-56 °C
1.5. (*S*)-8,8′-dithiobis(3-méthyl-1,2,3,4-tétrahydroquinoléine)
   On reprend 1,4 g (6,8 mmoles) du composé obtenu dans l'étape précédente dans 14 ml de potasse alcoolique 3 N et on chauffe au reflux pendant 6 heures. On verse le milieu réactionnel sur 50 ml d'eau et on ajuste le pH du mélange à 4-5 avec de l'acide chlorhydrique. On extrait le mélange avec de l'éther, on recueille la phase éthérée, on la sèche sur sulfate de magnésium et on évapore à sec. On reprend le résidu dans 50 ml de benzène et on ajoute 10 g d'alumine. On agite à l'air pendant 18 heures, on filtre l'alumine et on la lave avec du dichlorométhane. On réunit les filtrats et on les évapore à sec. On purifie le résidu par chromatographie sur colonne de gel de silice en éluant par un mélange cyclohexane:acétate d'éthyle (95:5).
   On obtient 0,8 g de produit jaune.
   Point de fusion = 78-80 °C
1.6. acide (*S*)-3-méthyl-1,2,3,4-tétrahydroquinoléine-8-sulfonique
   On dissout 0,8 g (4,5 mmoles) du composé obtenu dans l'étape précédente dans 4,51 g d'une solution d'acide sulfurique à 95 %, on place le milieu réactionnel dans un bain de glace et on ajoute lentement 1,49 ml d'eau oxygénée à 35 %. Ensuite on ajoute au milieu réactionnel de l'eau puis de la glace et on filtre le précipité ainsi obtenu. On le lave successivement avec 5 ml d'eau glacée, 3 fois 20 ml d'éther puis 10 ml de méthanol glacé. Finalement on rince à l'éther et on sèche à l'étuve sous pression réduite.
   On obtient 0,5 g de produit.
   Point de fusion = 255 °C (décomposition)
   [α]_{D}= + 38 ° [c = 0,2; méthanol:eau (50:50)]

### Exemple 2

chlorure de l'acide (*S*)-3-méthyl-1,2,3,4-tétrahydroquinoléine-8-sulfonique

On dissout 1,07 g de triphénylphosphine dans 7,5 ml de dichlorométhane à 0 °C. Sous atmosphère d'azote, à 0 °C, on ajoute goutte à goutte 0,3 ml de chlorure de sulfuryle puis on laisse le mélange remonter à la température ambiante. On ajoute ensuite lentement une solution contenant 0,47 g (2,1 mmoles) de l'acide obtenu dans l'étape précédente, 0,3 ml de triéthylamine et 12 ml de dichlorométhane. On agite pendant 1 heure à la température ambiante puis on verse le milieu réactionnel sur 200 ml de pentane. On le filtre, on évapore le filtrat à sec, on reprend le résidu dans 100 ml de pentane et on évapore à sec.
On obtient 0,4 g de produit utilisé tel quel dans l'étape suivante.

### Exemple 3

acide (*R*)-3-méthyl-1,2,3,4-tétrahydroquinoléine-8-sulfonique

A partir du (*S*)-3-iodo-2-méthylpropanoate de méthyle, selon le procédé décrit dans l'exemple 1, on obtient l'acide (*R*)-3-méthyl-1,2,3,4-tétrahydroquinoléine-8-sulfonique.
Point de fusion = 255 °C (décomposition)
[α]_{D}= - 39 ° [c = 0,2; méthanol:eau (50:50)]

### Exemple 4

chlorure de l'acide (*R*)-3-méthyl-1,2,3,4-tétrahydroquinoléine-8-sulfonique

A partir du composé obtenu dans l'exemple 3 et selon le procédé décrit dans l'exemple 2, on obtient le chlorure de l'acide (*R*)-3-méthyl-1,2,3,4-tétrahydroquinoléine-8-sulfonique.

Les composés de l'invention sont utiles pour la synthèse de composés à activité antithrombotique tels que ceux décrits dans le brevet européen EP 0008746, comme par exemple l'argatroban, et tels que les composés de formule (1) 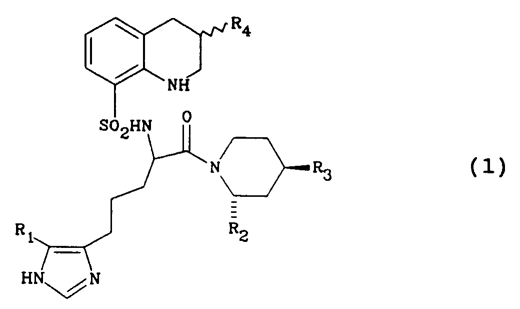
dans laquelle
R₁ représente soit un atome d'hydrogène, soit un groupe (C₁-C₄)alkyle,
R₂ représente divers substituants,
R₃ représente un groupe (C₁-C₄)alkyle et
R₄ est tel que défini précédemment.

Des composés de formule (1) dans laquelle R₂ représente soit un atome d'hydrogène, soit un groupe (C₁-C₄)alcoxycarbonyle, soit un groupe carboxylique, soit un groupe carboxylate de sodium, soit un groupe -CH₂OR₄ où R₄ est un atome d'hydrogène, un groupe (C₁-C₄)alkyle ou (C₁-C₄)acyle, soit un groupe -CONR₅R₆, soit un groupe -CN₄R₅ où R₅ est un atome d'hydrogène ou un groupe (C₁-C₄)alkyle et R₆ est un atome d'hydrogène, un groupe (C₁-C₄)alkyle ou hydroxy ou (C₁-C₄)alcoxy ou (C₁-C₃) alcoxyphényle sont décrits dans la demande de brevet européen EP 0565396.

La synthèse des composés de formule (1) à partir des composés selon l'invention est décrite dans le schéma 2.
On fait réagir un composé de formule (Ib) dans laquelle 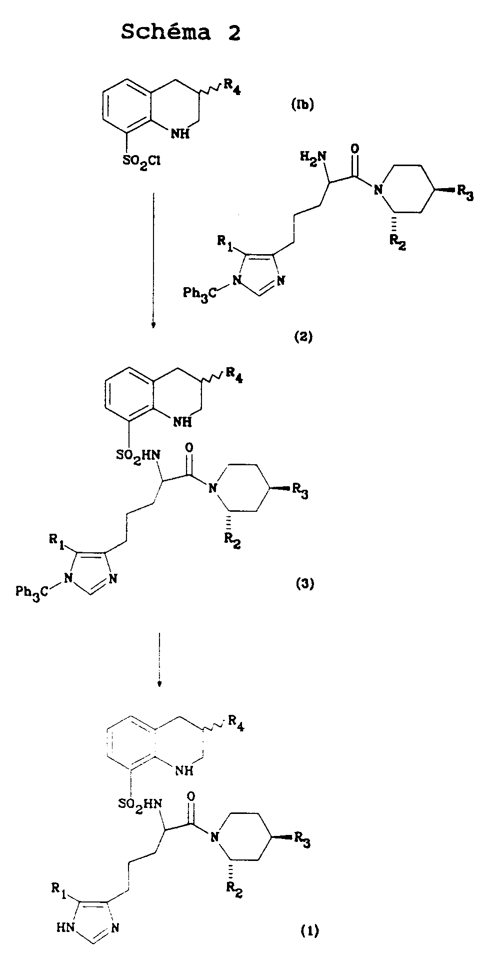
R₄ est tel que défini précédemment avec un composé de formule (2) dans laquelle R₁, R₂ et R₃ sont tels que définis précédemment pour obtenir un composé de formule (3) que l'on déprotège à chaud en présence d'acide acétique.

L'exemple A illustre la synthèse de composés de formule (1) à partir des composés selon l'invention.

### Exemple A

2*R*-[1(2*S*, 3*S*), 2α, 4β-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine-2-méthanol
A.1. acétate de [2*R*-[1(2*S*, 3*S*), 2α, 4β]]-[4-méthyl-1-[2-[[(3-méthyl-1,2,3,4-tétrahydroquinoléin-8-yl)sulfonyl]amino]-1-oxo-5-[1-(triphénylméthyl)-1*H*-imidazol-4-yl]pentyl]pipéridin-2-yl]méthyle
   A.1.1. chlorhydrate de l'acétate de [2*R*-[1(2*S*), 2α, 4β]]-[1-2-amino-1-oxo-5-[1-(triphénylméthyl)-1*H*-imidazol-4-yl]pentyl]-4-méthylpipéridin-2-yl]méthyle
      A.1.1.1. chlorure de triphényl[[(1-triphénylméthyl)-1*H*-imidazol-4-yl]méthyl]phosphonium
         A 670 ml d'une solution de 105,5 g (294 mmoles) de 4-(chlorométhyl)-1-(triphénylméthyl)-1*H*-imidazole dans le diméthylformamide, on ajoute 77,7 g (296 mmoles) de triphénylphosphine. On chauffe à 80 °C pendant 3 heures. On évapore le solvant, on reprend le brut dans l'éther et on le triture. On filtre le précipité et on le sèche sous vide sur pentoxyde de phosphore.
         On obtient 162 g de produit sous forme de cristaux jaunâtres.
         Point de fusion = 210 °C Rendement = 89 %
      A.1.1.2. (*S*,*E*)-2-[[(phénylméthoxy)carbonyl]amino]-5-[1-(triphénylméthyl)-1*H*-imidazol-4-yl]pent-4-énoate de 1,1-diméthyléthyle
         Dans un ballon tricol, sous argon, on introduit 50,93 g (820 mmoles) de chlorure de triphényl[[1-triphénylméthyl)-1*H*-imidazol-4-yl]méthyl]phosphonium en solution dans 333 ml de tétrahydrofurane. On ajoute goutte à goutte, à - 70 °C, 51,2 ml d'une solution 1,6 M de n-butyllithium dans l'hexane (820 mmoles). Aprés 30 minutes d'agitation à - 70 °C, on verse rapidement le milieu réactionnel dans 270 ml d'une solution 0,253 M, refroidie à -70 °C, de (*S*)-4-oxo-2-[[(phénylméthoxy)carbonyl]amino]butanoate de 1,1-diméthyléthyle dans le tétrahydrofurane (683 mmoles). On laisse remonter le mélange à la température ambiante pendant une nuit. On hydrolyse le mélange avec 280 ml d'une solution aqueuse saturée de chlorure de sodium. On sépare la phase aqueuse de la phase organique et on l'extrait par 2 fois 140 ml d'acétate d'éthyle. On réunit les phases organiques, on les sèche sur du sulfate de magnésium et on évapore à sec. On purifie par chromatographie sur colonne de gel de silice en éluant par un gradient hexane/acétate d'éthyle.
         On obtient un mélange d'oléfine cis et trans.
         Pour la forme cis:
         Point de fusion = 66 °C
         R_{f} = 0,30 [hexane/acétate d'éthyle (60/40)]
         Pour la forme trans:
         R_{f} = 0,15 [hexane/acétate d'éthyle (60/40)]
         Rendement = 40 %
      A.1.1.3. acide (*S*,*E*)-2-[[(phénylméthoxy)carbonyl]amino]-5-[1-(triphénylméthyl)-1*H*-imidazol-4-yl]-pent-4-ènoïque
         On place 3,9 g (6,37 mmoles) du composé trans obtenu dans l'étape précédente dans 80 ml de benzène puis on fait passer à 0 °C un courant d'acide chlorhydrique gazeux jusqu'à saturation. Ensuite on laisse sous agitation pendant 4 heures à la température ambiante puis on évapore à sec. On reprend le résidu par 20 ml de dichlorométhane, on le neutralise par de l'ammoniac et on le purifie par chromatographie sur colonne de gel de silice en éluant par un mélange dichlorométhane/méthanol (90:10).
         On obtient 3 g de produit.
         Point de fusion = 180 °C (décomposition)
      A.1.1.4. [2*R*-[1(1*S*), 2α, 4β]]-[1-[[2-(acétyloxy)méthyl]-4-méthyl-pipéridin-1-yl]carbonyl]-4-[1-(triphénylméthyl)-1*H*-imidazol-4-yl]but-3-ényl]carbamate de phénylméthyle
         A une solution de 1,14 g (3 mmoles) de trifluoroacétate d'acétate de (2*R*-*trans*)-(4-méthylpipéridin-2-yl)méthyle dans 20 ml de dichlorométhane, on ajoute à 0 °C, 1,67 g (3 mmoles) du composé obtenu dans l'étape précédente, 1,5 ml de N,N-diisopropyléthylamine puis 1,5 g (3,3 mmoles) d'hexafluorophosphate de [(benzotriazol-1-yl)oxy]tris(diméthylamino) phosphonium. On laisse le mélange pendant une nuit à la température ambiante puis on le verse sur 100 ml d'acétate d'éthyle. On lave successivement par 100 ml d'une solution 0,1 N d'acide chlorhydrique, 100 ml d'une solution saturée d'hydrogénocarbonate de sodium, 100 ml d'une solution saturée de chlorure de sodium puis on sèche sur sulfate de magnésium et on évapore à sec.
         On obtient 1,4 g de produit sous forme d'un solide.
         Point de fusion = 59 °C
      A.1.1.5. chlorhydrate de l'acétate de [2*R*-[1(2*S*), 2α, 4β]]-[1-[2-amino-1-oxo-5-[1-(triphénylméthyl)-1*H*-imidazol-4-yl]pentyl]-4-méthylpipéridin-2-yl]méthyle
         Dans une fiole de Parr, on place 1,3 g (1,8 mmoles) du composé obtenu dans l'étape précédente et on ajoute 30 ml d'éthanol et 0,4 g de palladium sur charbon à 10 %. On hydrogène à 50 psi pendant 8 heures puis on filtre et on essore le catalyseur. On recueille le filtrat et on l'évapore à sec. On reprend le résidu dans 20 ml d'isopropanol chlorhydrique 0,1 N puis on évapore à sec.
         On obtient 1,12 g de produit sous forme de chlorhydrate.
         R_{f} = 0,55 [méthylisobutylcétone/acide acétique/eau (60/20/20)]
   A.1.2. acétate de [2*R*-[1(2*S*, 3*S*), 2α, 4β-[4-méthyl-1-[2-[[(3-méthyl-1,2,3,4-tétrahydroquinoléin-8-yl)sulfonyl] amino]-1-oxo-5-[1-(triphénylméthyl)-1*H*-imidazol-4-yl]pentyl]pipéridin-2-yl]méthyle
      A 0,6 g (0,9 mmoles) de chlorhydrate de l'acétate de [2*R*-[1(2*S*), 2α, 4β]]-[1[2-amino-1-oxo-5-[1-(triphénylméthyl)-1*H*-imidazol-4-yl]pen-tyl]-4-méthylpipéridin-2-yl]méthyle obtenu selon la méthode décrite précédemment, on ajoute, à 0 °C, 0,4 g du composé obtenu dans l'exemple 2 en solution dans 10 ml de dichlorométhane et 0,38 ml de triéthylamine dans 10 ml de dichlorométhane. On laisse sous agitation pendant une nuit à la température ambiante puis on lave successivement le mélange par 10 ml d'une solution 0,2 N d'acide chlorhydrique, 10 ml d'une solution saturée d'hydrogénocarbonate de sodium et 10 ml d'une solution saturée de chlorure de sodium. On filtre le milieu réactionnel, on le sèche sur sulfate de magnésium et on évapore à sec. On purifie le résidu par chromatographie sur colonne de gel de silice en éluant par un mélange dichlorométhane:éthanol (96:4).
      On obtient 0,96 g de produit.
      Point de fusion = 49 °C.
A.2. acétate de [2*R*-[1(2*S*, 3*S*), 2α, 4β]]-1-[5-(1*H*-imidazol-4-yl)-2-[[(3-méthyl-1,2,3,4-tétrahydroquinoléin-8-yl) sulfonyl]amino]-1-oxopentyl]-4-méthylpipéridine-2-yl] méthyle
   On place 0,65 g (0,74 mmoles) du composé obtenu dans l'étape précédente dans 10 ml d'acide acétique. On ajoute 2 ml d'eau et 8 ml de tétrahydrofurane puis on chauffe à 80 °C pendant 1 heure. On évapore le milieu réactionnel à sec et on purifie le résidu par chromatographie sur colonne de gel de silice en éluant par un mélange dichlorométhane:éthanol (90:10).
   On obtient 0,4 g de produit pur.
   Point de fusion = 47 °C
A.3. 2*R*-[1(2*S*, 3*S*), 2α, 4β]]-1-[5-(1*H*-imidazol-4-yl)-2-[[(3-méthyl-1,2,3,4-tétrahydroquinoléin-8-yl)sulfonyl]amino)-1-oxopentyl]-4-méthylpipéridine-2-méthanol
   On place 0,4 g (0,75 mmoles) du composé obtenu dans l'étape précédente dans 2 ml de méthanol, on refroidit à 0 °C et on ajoute goutte à goutte 1,5 ml d'une solution 1 N de soude. On laisse sous agitation pendant 2 heures à 0 °C, on évapore le méthanol et on suspend le résidu dans un mélange dichlorométhane:eau (50:50). On récupère la phase organique que l'on sèche sur sulfate de magnésium et que l'on évapore à sec. On reprend le résidu par un équivalent d'une solution 1 N d'acide chlorhydrique et on le purifie par chromatographie sur colonne de gel de silice en éluant par un gradient d'acide chlorhydrique 0,01 N et d'acétonitrile (0 à 100 % en acétonitrile). On rassemble les fractions contenant le produit et on les lyophilise.
   On obtient 0,3 g de produit.
   Point de fusion = 90 °C
   [α]_{D} = + 110 ° (c = 0,2; méthanol)

Les composés de formule (1) ainsi obtenus présentent des propriétés antithrombotiques et sont décrits dans la demande de brevet européen EP 0565396 de la demanderesse.

## Revendications

1. Dérivés optiquement purs répondant à la formule (I) 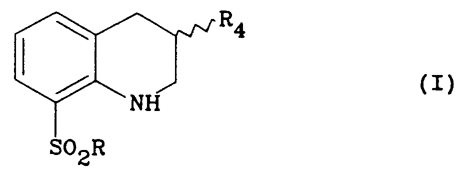 dans laquelle
R représente soit un atome de chlore soit un groupe hydroxy et
R₄ représente un groupe (C₁-C₄)alkyle.

2. L'acide (*S*)-3-méthyl-1,2,3,4-tétrahydroquinoléine-8-sulfonique.

3. Le chlorure de l'acide (*S*)-3-méthyl-1,2,3,4-tétrahydroquinoléine-8-sulfonique.

4. L'acide (*R*)-3-méthyl-1,2,3,4-tétrahydroquinoléine-8-sulfonique.

5. Le chlorure de l'acide (*R*)-3-méthyl-1,2,3,4-tétrahydroquinoléine-8-sulfonique.

6. Procédé de préparation des composés selon la revendication 1, procédé caractérisé en ce que l'on fait réagir un composé de formule (II) 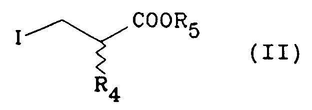 (dans laquelle R₄ est tel que défini ci-dessus et R₅ représente un groupe (C₁-C₄)alkyle) avec du 1-iodo-2-nitrobenzène en présence d'un catalyseur dans un solvant aprotique et on obtient un composé de formule (IV) 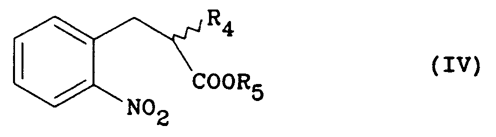 que l'on soumet à une hydrogénation catalytique, pour obtenir une dihydroquinoléine de formule (V) 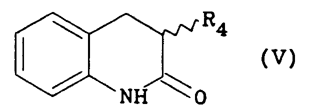 que l'on fait réagir avec du borane dans un solvant aprotique obtenant ainsi une tétrahydroquinoléine de formule (VI) 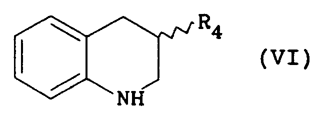 que l'on fait réagir avec du chlorure de chlorocarbonylsulfényle dans un solvant aprotique,
obtenant ainsi un composé de formule (VII) 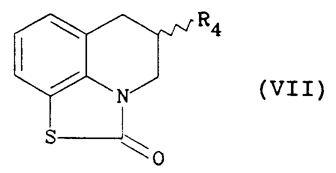 que l'on traite par de la potasse alcoolique puis par de l'alumine
pour obtenir un composé de formule (VIII) 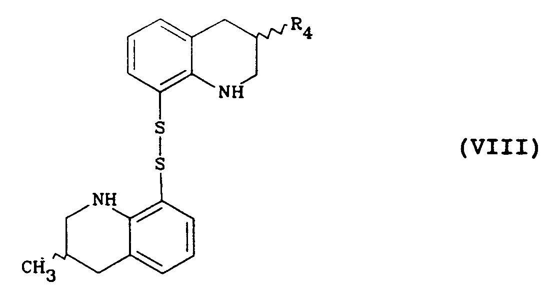 que l'on traite par de l'eau oxygénée en présence d'acide sulfurique pour obtenir l'acide sulfonique de formule (Ia) 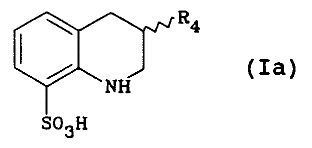 qui correspond à la formule (I) lorsque R représente un groupe hydroxy,
puis pour préparer les composés de formule (I) dans laquelle R représente un atome de chlore, on fait réagir le composé de formule (Ia) sur du chlorure de sulfuryle en présence de triphénylphosphine et d'une base dans un solvant aprotique obtenant ainsi un composé de formule (Ib)

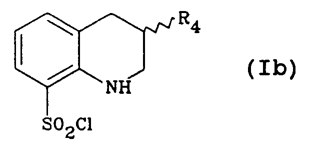

7. Procédé selon la revendication 6 caractérisé en ce que le composé de formule (II) est le (*R*)-3-iodo-2-méthylpropano-ate de méthyle.

8. Procédé selon la revendication 6 caractérisé en ce que le composé de formule (II) est le (*S*)-3-iodo-2-méthylpropano-ate de méthyle.

9. Utilisation des composés selon la revendication 1 pour la préparation de composés à activité antithrombotique.
